# EUROPEAN PATENT APPLICATION

(11) **EP 3 059 593 A1**
(43) Date of publication of application: **24.08.2016**
(21) Application number: 14854826.6
(22) Date of filing: 18.09.2014
(51) Int. Cl.: G01N 33/68, G01N 33/53, G01N 27/62, G01N 30/72

(54) **MASS SPECTROMETRY OF BRAIN NATRIURETIC PEPTIDE IN BLOOD USING CHEMICAL ISOTOPIC SUBSTITUTION**

(30) Priority: 16.10.2013 KR 20130123364
(71) Applicant: Korea Research Institute of Standards and Science, Daejeon 305-340 (KR)
(72) Inventor: KANG, Dukjin, Daejeon 302-847 (KR); KIM, Sook-Kyung, Daejeon 305-315 (KR); LEE, Sun Young, Daejeon 305-340 (KR)
(74) Representative: dompatent von Kreisler Selting Werner - Partnerschaft von Patent- und Rechtsanwälten mbB
(86) International application number: PCT/KR2014/008684
(87) International publication number: WO 2015/056889

(57) **Abstract**

The present invention relates to a quantitative analysis method on the basis of an isotope-dilution mass spectrometry using a chemical isotope labelling method for a brain natriuretic peptide which is widely used as a biomarker for diagnosis and prognostic evaluation of cardiovascular-associated diseases. The quantitative analysis method of a brain natriuretic peptide in blood using a chemical isotope labelling technique is a brain natriuretic peptide-specific quantitative analysis technique which is more accurate than a conventional immunoassay using an antibody.

## Description

### [Technical Field]

The present invention relates to a mass spectrometry specific to a brain natriuretic peptide which is a cardiovascular disease diagnostic biomarker, using chemical isotopic substitution, and a method for determining occurrence of the cardiovascular disease and evaluating a prognosis thereof through experimental results obtained by the mass spectrometry.

### [Background Art]

In accordance with the recent domestic and international development of analysis techniques converged with tandem mass spectrometry (MS/MS), research into characteristics of various proteins present in cells and research into proteomics capable of conducting qualitative and quantitative analysis have been actively conducted. In particular, these researches aim to define causes of diseases associated with improvement of the quality of human life at protein levels. Particularly, it is possible to conduct quantitative comparison of protein samples between normal subjects and patients through development of a protein-specific and stable isotope-labeling method with regard to a biological sample such as cells, tissue, blood serums, etc., which contribute to the discovery of biomarkers for early diagnosis of various diseases and clinical diagnosis fields. However, the quantitative analysis of proteins using the stable isotope-labeling method has a problem in that cell-related experts or amino acids substituted with high-priced isotopes and complicated pre-treatment techniques such as SILAC (stable isotope labeling with amino acids in cell culture), MeCAT (metal-coded affinity tags), TMT (tandem mass tags), etc., are required.

Due to the above problem, an immunoassay using a protein marker-specific antibody has been the most widely used in most of the clinical diagnosis evaluation. In particular, for diagnosis and prognostic evaluation of cardiovascular diseases, quantitative changes of a brain natriuretic peptide (BNP) protein marker are diagnosed through the immunoassay. However, there are disadvantages in that uncertainty of quantitative measurement of the brain natriuretic peptide is large due to low reproducibility of antigen-antibody non-specific reaction and a fluorescein labeling technique of an immunoassay, high-priced enzymes are used, long periods of reaction time are needed, etc., which is not effective in conducting the diagnosis and the prognostic evaluation, and as a result, another alternative is required.

Therefore, the present inventors recognized the need for development of a sample pre-treatment technique and quantitative analysis that are more accurately and easily conducted for early detection and verification of cardiovascular diseases, developed mass spectrometry of brain natriuretic peptide in blood using chemical isotopic substitution, and completed the present invention.

### [Disclosure]

### [Technical Problem]

An object of the present invention is to provide an isotope-coded carbamidomethylation (iCCM) isotope labeling capable of solving a antigen-antibody non-specific reaction and low reproducibility of fluorescein labeling technique of an immunoassay, and simultaneously solving a complicated sample pre-treatment process of an isotope labeling technique, and a quantitative analysis method specific to a brain natriuretic peptide on the basis of a mass spectrometer using the same.

### [Technical Solution]

In one general aspect, a mass spectrometry includes: a) adding and reacting a protein reducing agent to a brain natriuretic peptide, Troponin I protein or Troponin T protein; b) reacting protein mixtures of step a) with iodine acetamide (IAA) and with an isotope of iodine acetamide, respectively; and c) obtaining mass spectra of two kinds of proteins obtained by step b) by using a mass spectrometer.

Troponin I is not found in normal skeletal muscle, but exists only in adult myocardium. Accordingly, under a situation in which creatine kinase isoenzymes (CK-MB) is increased in addition to myocardial damage, Troponin I is not detected in blood, which is very specific to the myocardial damage, and is associated with a myocardial infarct size. In addition, it is known that when acute cardiovascular diseases occur, an expression level thereof is rapidly increased, and detection of this protein is significantly important for early diagnosis of the acute cardiovascular diseases. Meanwhile, when myocardial damage occurs, Troponin T is continuously dissociated in blood, and a concentration thereof is increased. After heart disease, Troponin T has the highest value within 24 hours, and is continuously present for 9 days or more as the highest value or more, such that diagnosis time thereof is broader than those of creatine kinase and creatine kinase isozyme. Accordingly, Troponin T has high sensitivity and specificity, which is useful for early diagnosis and subsequent diagnosis of myocardial infarction and is advantageous to estimate an infarct size.

The protein reducing agent in step a) is not limited, but for example, dithiothreitol, dithioerythritol, tris(2-carboxyethyl)phosphine, or tributylphosphine may be used as the protein reducing agent. Preferably, dithiothreitol may be used. The reacting in step b) may change reactivity of a thiol group of cysteine into an irreversible form in order to prevent cysteine-cysteine refolding. For improving the enzyme treatment with regard to the protein in the enzyme treatment process, disulfide bonds formed by cysteine-cysteine bonds of the protein are firstly subjected to protein denaturation by using the reducing agent such as dithiothreitol (DTT), and iodine acetamide which is an alklyating agent for a thiol group (-SH) of the cysteine is added in order to prevent the cysteine-cysteine refolding, such that reactivity of the thiol group of the cysteine is changed into the irreversible form. In accordance with the chemical modification by iodine acetamide, improvement in the efficiency of enzyme treatment with regard to protein samples and reproducibility may be finally secured. The cysteine-specific CM (carbamidomethylation) process by iodine acetamide results in an increase of 57.05 Da to the cysteine group. The present invention is characterized by an isotope labeling method for BNP quantitative analysis using the CM-based chemical modification.

The isotope of iodine acetamide in step b) is not limited, but may be iodine acetamide-¹³C₂, D₂. In order to conduct the isotope labeling for BNP quantitative analysis, iodine acetamide and the isotope of iodine acetamide, i.e., iodine acetamide-¹³C₂, D₂ are reacted with two standard BNP protein solutions each having a purity of 97% or more, respectively. Accordingly, a molecular weight of the BNP having two cysteine groups is increased by two spots of CM reactions. Finally, BNP-CM₂ and BNP-iCCM₂ produced by iodine acetamide and the isotope of iodine acetamide, i.e., iodine acetamide-¹³C₂, D₂ have molecular weights of 3578.12 Da and 3586.12 Da, respectively. That is, a difference in molecular weight between two BNP protein samples may be 8 Da. Therefore, a difference in molecular weight between the iodine acetamide and the isotope of iodine acetamide may be preferably 3 to 5 Da.

The mass spectrometer of step c) is not limited, but for example, a liquid chromatography-electrospray ionization-tandem mass spectrometry (LC-ESI-MS/MS) may be used.

Further, in another general aspect, a method for diagnosis and prognostic evaluation of cardiovascular diseases includes: steps a) to c) as described above; and d) determining whether the protein and an isotope of protein are appropriate as a material for diagnosis or prognostic evaluation of cardiovascular diseases, from the mass spectra obtained by step c).

### [Advantageous Effects]

The quantitative analysis method of the brain natriuretic peptide according to the present invention is capable of minimizing time loss and economic loss caused from quantitative analysis by a high-priced isotope-substituted peptide labeling pre-treatment method or a complicated protein isotope labeling pre-treatment method, and is capable of reducing uncertainty in quantitative measurement and side reactions caused during the protein isotope labeling pre-treatment method. According to the quantitative analysis method on the basis of the brain natriuretic peptide isotope-labeling using iCCM as described in the present invention, low-priced iodine acetamide (IAA) and iodine acetamide-¹³C₂, D₂ may be used, the isotope labeling pre-treatment method for quantitative analysis of the brain natriuretic peptide protein may be performed within 30 minutes, which is significantly applicable to a more accurate diagnosis for cardiovascular diseases as compared to a conventional quantitative analysis method.

### [Description of Drawings]

FIG. 1 shows an overview of a carbamidomethylation (CM) reaction of a brain natriuretic peptide (BNP) protein using iodine acetamide (IAA), and analysis results through LC-ESI-FT orbitrap-MS.
FIG. 2 shows structural difference of BNP-CM₂ and BNP-iCCM₂ using CM-based IAA and isotope IAA-¹³C₂, D₂ and molecular weights thereof measured through mass analysis.
FIG. 3 shows base peak chromatogram (BPC) and mass spectrum of a mixture of BNP-CM₂ and BNP-iCCM₂, detected through LC-ESI-FT orbitrap-MS analysis.
FIG. 4 shows base peak chromatogram (BPC) and BNP-CM₂-specific and BNP-iCCM₂-specific mass spectra with regard to a blood serum sample to which 400 pg BNP-iCCM₂ is added.
FIG. 5 shows BNP-CM₂-specific and BNP-iCCM₂-specific extracted ion chromatogram (XIC) analysis results with regard to a blood serum sample to which 400 pg BNP-iCCM₂ is added.

### [Best Mode]

Hereinafter, exemplary embodiments of the present invention will be described in detail with reference to the following Examples and the accompanying drawings. However, the detailed description is to help a specific understanding of the present invention, and the protection scope of the present invention is not limited to Examples below.

### (Example 1) Brain natriuretic peptide (BNP)-specific carbamidomethylation (CM) and mass spectrometer (MS) spectrum using iodine acetamide (IAA)

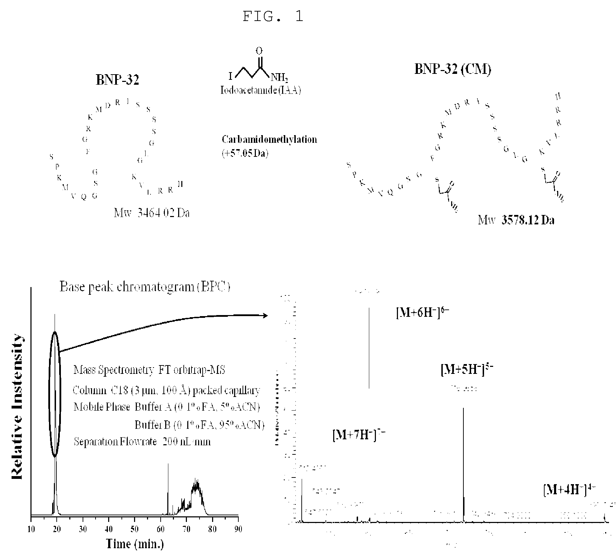

BNP (molecular weight of 3464.02 Da), DTT, IAA, and its isotope IAA-¹³C₂, D₂ were purchased from Sigma-Aldrich®. In order to verify CM reactivity and label efficiency with regard to a standard BNP protein, BNP protein (1 mg) was dissolved in 50 mM NH₄CO₃ (1 mL) solution, and DTT (1 mg) was added thereto, followed by gently shaking and reacting at 37°C for 2 hours. 40 mM IAA solution (20 µL) was added to the NH₄CO₃ solution, and reacted in a darkroom at room temperature for 30 minutes. Mass spectra with regard to the BNP-CM₂ protein obtained by the cysteine-specific CM process by the IAA was examined by using FT orbitrap-MS, and schematized in FIG. 1.

FIG. 1 shows BPC (base peak chromatogram) and mass spectrum detected at 19.2 minutes of the BNP protein (1 ng) obtained by using FT orbitrap-MS, after the CM-pretreated standard BNP protein (1 ng) was separated by using a capillary tube (internal diameter of 75 µm and external diameter of 360 µm) filled with C8(5 µm, 200 Å). It was confirmed that 99% or more of BNP-CM₂ having a molecular weight of 3578.12 Da was detected, which is 114.10 Da larger than a unique molecular weight of the BNP protein, i.e., 3464.02 Da, in accordance with two CM reactions. In particular, multiple hydride ions such as [M+4H⁺]⁴⁺, [M+5H⁺]⁵⁺, [M+6H⁺]⁶⁺, [M+7H⁺]⁷⁺, etc., could be confirmed.

### (Example 2) Comparison in efficiency between BNP-specific CM and BNP-specific iCCM isotope-labeling using IAA and isotope IAA-¹³C₂, D₂

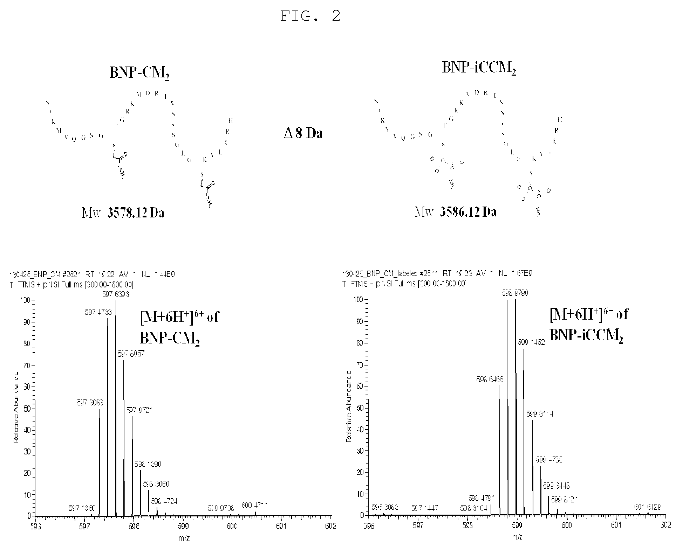

BNP-CM₂ and BNP-iCCM₂ each having different molecular weight were prepared through CM reaction using IAA and IAA-¹³C₂, D₂ on the BNP proteins, respectively, and mass spectra of the BNP-CM₂ and the BNP-iCCM₂ obtained by using a mass spectrometer were shown in FIG. 2.

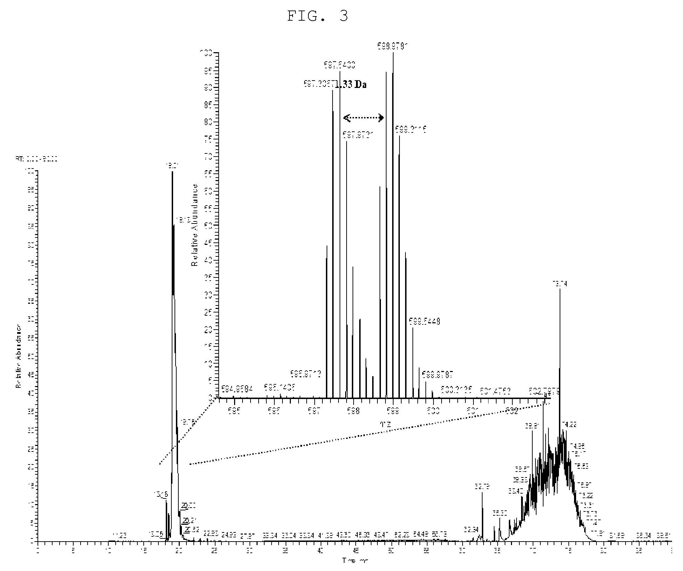

BNP-CM₂ produced by the IAA and BNP-iCCM₂ produced by the isotope IAA-¹³C₂, D₂ were mixed at a weight ratio of 1:1, and BPC was measured by LC-ESI-FT orbitrap-MS analysis method, and mass spectrum obtained by elution at 19 minutes was secured, and shown in FIG. 3.

As a result of comparison with mass/charge amount values, i.e., m/z values with regard to [M+6H⁺]⁶⁺ ion among precursor ions for BNP-CM₂(3578.12 Da) and BNP-iCCM₂(3586.12 Da) produced by IAA as shown in FIG. 2, it could be confirmed that the m/z values of the BNP-CM₂(3578.12 Da) and BNP-iCCM₂ (3586.12 Da) were 597.6393 and 598.9790, respectively, and a difference between two proteins was about 1.333 Da (+6). Based on the result above, it was confirmed that the difference in molecular weight between two proteins was 8 Da, which is the same efficiency as the result by the CM reaction. In particular, BNP-CM₂ products of which a m/z value shown by IAA is 597.6393 was not found like the mass spectrum of BNP-iCCM₂ using IAA-¹³C₂, D₂, from which it indicates that an efficiency of producing the isotope labeling material of BNP-iCCM₂ was made close to 100%.

As shown in FIG. 3, a difference in m/z between the BNP-CM₂ protein and the BNP-iCCM₂ protein in a state in which two of the proteins were mixed with each other was 1.333 Da. In addition, quantitative changes of two of the proteins were not found even in the mixing pre-treatment process of the protein produced by the different chemical reactions, from which it could be appreciated that this method is an isotope labeling method that is able to applicable to a quantitative comparative study on protein mixture samples present in various media such as blood serum, urine, etc., without mutual side reactions.

### (Example 3) Quantitative analysis for blood serum BNP protein using CM-based isotope labeling method

BNP protein actually included in a blood serum sample was quantified on the basis of the BNP-specific isotope labeling pre-treatment methods of Examples 1 and 2. The blood serum sample (500 µL) of a cardiovascular patient was CM-treated by IAA, and mixed with 400 pg BNP-iCCM₂. Then, BPC and mass spectrum of the sample eluted by a solution in which ACN:H₂O is mixed at a ratio of 10:90 (v/v) using C18 SPE cartridge were secured by LC-ESI-FT orbitrap-MS.

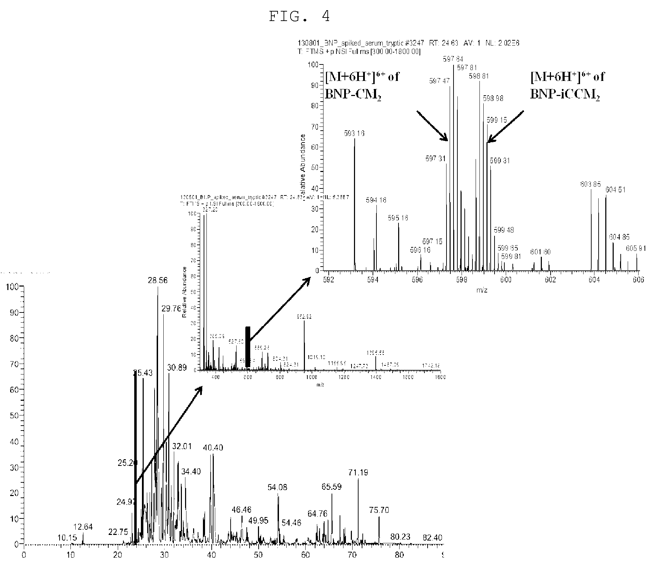

It was confirmed that mixtures of proteins and peptides in various shapes were detected as shown in FIG. 4. Particularly, within a range at which m/z is 592 to 606 in mass analysis spectrum detected at 24 minutes, ions of which m/z values produced by BNP-CM₂ and BNP-iCCM₂ are 597.6393 and 598.9790, respectively, were confirmed.

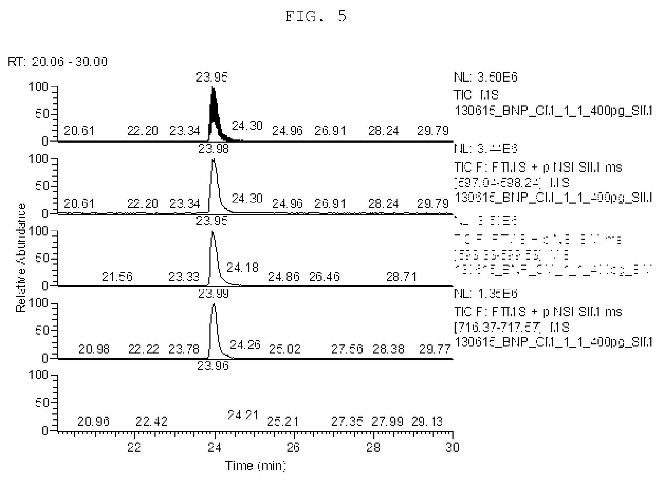

FIG. 5 shows extracted ion chromatogram (XIC) obtained in a single ion observation (SIM) mode with respect to ions of which the m/z values detected by BNP-CM₂ present in blood serum are 597.6393 (+6) and 716.9556 (+5), and the m/z values shown by 400 pg BNP-iCCM₂ added to the blood serum are 598.9790 (+6) and 718.5556 (+5). On the basis of results of FIG. 5, it was confirmed that according to the mass spectrometry of the present invention, quantitative evaluation with regard to the BNP protein actually present in blood serum was possibly performed through quantitative information of isotope-labeled BNP-iCCM₂ protein. In addition, it was confirmed that the mass spectrometry of the present invention is significantly applicable to isotope-dilution mass spectrometry (ID-MS) which is the top-level quantitative analysis method.

## Claims

1. A mass spectrometry comprising:
a) adding and reacting a protein reducing agent to a brain natriuretic peptide, Troponin I protein or Troponin T protein;
b) reacting protein mixtures of step a) with iodine acetamide and with an isotope of iodine acetamide, respectively; and
c) obtaining mass spectra of two kinds of proteins obtained by step b) by using a mass spectrometer.

2. The mass spectrometry of claim 1, wherein the protein reducing agent in step a) is dithiothreitol, dithioerythritol, tris(2-carboxyethyl)phosphine or tributylphosphine.

3. The mass spectrometry of claim 1, wherein the reacting in step b) changes reactivity of a thiol group of cysteine into an irreversible form in order to prevent cysteine-cysteine refolding.

4. The mass spectrometry of claim 1, wherein the isotope of iodine acetamide in step b) is iodine acetamide-¹³C₂, D₂.

5. The mass spectrometry of claim 4, wherein a difference in molecular weight between the iodine acetamide and the isotope of iodine acetamide is 3 to 5 Da.

6. The mass spectrometry of claim 1, wherein the mass spectrometer of step c) is LC-ESI-MS/MS.

7. A method for diagnosis and prognostic evaluation of cardiovascular diseases comprising:
steps a) to c) of claim 1; and
d) determining whether the protein and an isotope of protein are appropriate as a material for diagnosis or prognostic evaluation of cardiovascular diseases, from the mass spectra obtained by step c).
